# EUROPEAN PATENT APPLICATION

(11) **EP 3 667 324 A1**
(43) Date of publication of application: **17.06.2020**
(21) Application number: 18910163.7
(22) Date of filing: 12.07.2018
(51) Int. Cl.: G01N 33/76

(54) **HCG CYCLE TEST PAPER STRIP, KIT, PREPARATION METHOD THEREFOR AND USE THEREOF**

(30) Priority: 12.03.2018 CN 201810204279
(71) Applicant: Nantong Egens Biotechnology Co., Ltd., Nantong, Jiangsu 226010 (CN)
(72) Inventor: OU, Weijun, Nantong, Jiangsu 226010 (CN); SUN, Yipin, Nantong, Jiangsu 226010 (CN); XU, Yan, Nantong, Jiangsu 226010 (CN)
(74) Representative: Lavoix
(86) International application number: PCT/CN2018/095410
(87) International publication number: WO 2019/174165

(57) **Abstract**

A hCG cycle test paper strip, comprising a first test paper strip (1) and a second test paper strip (2). The first test paper strip (1) and the second test paper strip (2) respectively comprise: a substrate and a sample pad, a colloidal gold adsorption pad, an antibody carrying film and a water adsorption pad adhered to the substrate in sequence. The antibody carrying film is provided with a detection line and a quality control line which are separated from each other. A colloidal gold-anti-human chorionic gonadotropin β-hCG monoclonal antibody conjugate is adsorbed on the colloidal gold adsorption pad (16) of the first test paper strip (1). A colloidal gold-anti-human chorionic gonadotropin β-hCG monoclonal antibody conjugate and a free β antibody are adsorbed on the colloidal gold adsorption pad (26) of the second test paper strip (2). The detection line is coated with α-hCG monoclonal antibody, and the control line is coated with anti-mouse IgG polyclonal antibody. The test paper strip can be used for a urine sample which does not need to be processed, the operation and detection method are simple, and thus the test paper is suitable for on-site, instant, and rapid detection and is suitable for the detection of the number of weeks of pregnancy in a family.

## Description

### CROSS REFERENCE TO RELATED APPLICATIONS

This application claims priority to Chinese Patent Application No. 201810204279.3 filed on March 12, 2018, entitled "hCG cycle test paper strip, kit, preparation method therefor and use thereof', the entire contents of which are incorporated herein by reference.

### TECHNICAL FIELD

The present application relates to a field of biological detection, and particularly relates to a hCG cycle test paper strip, kit, preparation method therefor and use thereof.

### BACKGROUND

Human chorionic gonadotropin (hCG) is a glycoprotein hormone secreted by placental trophoblast cells during pregnancy. It consists of two different subunits, α and β connected by a non-covalent bond. During production, secretion, and metabolism of hormones, hCG molecules will undergo various changes such as rupture and dissociation, so as to exist in various molecular forms in blood and urine. hCG is the only placenta hormone that does not increase with the increase in placental weight. Reference values of hCG of pregnant women are described in *"Chinese Clinical Test Operating Procedures".* The reference value of hCG is 5-50 IU/ml for 0.2-1 week of pregnancy, the reference value of hCG is 50-500 IU/ml for 1-2 weeks of pregnancy, the reference value of hCG is 100-5000 IU/ml for 2-3 weeks of pregnancy, the reference value of hCG is 500-10000 IU/ml for 3-4 weeks of pregnancy, the reference value of hCG is 1000-50,000 IU/ml for 4-5 weeks of pregnancy, the reference value of hCG is 10,000-100,000 IU/ml for 5-6 weeks of pregnancy, the reference value of hCG is 15,000-200,000 IU/m for 6-8 weeks of pregnancy, the reference value of hCG is 10,000-100,000 IU/ml for 8-12 weeks of pregnancy. A concentration of hCG is 5-50 mmIU/ml in the blood and >25 mIU/ml in the urine after 1 week of pregnancy, reaches a peak around 8-10 weeks, and decreases rapidly after the next 1-2 weeks, and then gradually decreases and maintains at about 1/5-1/10 of the peak level until delivery. Therefore, early pregnancy can be detected by detecting the hCG concentration in blood and urine.

At present, a test paper in a general early pregnancy test pen is mostly used by a double-antibody sandwich method, which uses colloidal gold as an indicator to detect the hCG concentration in urine, and can only be used to determine whether pregnancy or not, but not a number of days of pregnancy. The number of days of pregnancy is calculated according to a traditional method. During the early pregnancy, the number of days of pregnancy starts from a first day after the last menstrual period. However, this method is not accurate. At present, the more accurate method for detecting the number of days of pregnancy is mainly blood test, ultrasonic detection, and combination of HCG detection and ultrasonic detection, which require professionals to obtain a detection result after a long period of testing, and have a complicated operation.

Chinese patent CN101294966A discloses an ectopic pregnancy and pregnant days colloid gold detection agent and manufacturing method thereof. It discloses a piece of detecting test paper strip and urine sample treatment solution; wherein, the detecting test paper consists of a base plate, a sample pad, a gold label anti-β-hCG monoclone antibody layer and a cellulose nitrate film overlapped with and adhered to a absorption point and connected to the base plate; α-hCG monoclone antibody line and a sheep anti-rat polyclonal antibody line are arranged on the cellulose nitrate film, and the α-hCG monoclone antibody line is close to the gold label anti-β-hCG monoclone antibody layer. When this detection paper strip is used to detect the number of days of pregnancy, it requires three test paper strips and a processing of urine sample, resulting in complicated operation method and detection method, thus this detection agent is not suitable for detecting the number of days of pregnancy in a family.

### SUMMARY

Therefore, in order to overcome defects that the colloidal gold detection agent in the prior art requires three test paper strips and processing of urine, to detect the number of days of pregnancy, resulting in complicated operation method and detection method. The present application provides a hCG cycle test paper strip, kit, preparation method therefor and use thereof, which need few test strips and can be used for a urine sample which does not need to be processed, the operation and detection method are simple, and thus being suitable for the detection of the number of days of pregnancy in a family.

Therefore, the present application provides a hCG cycle test paper strip, comprising a first test paper strip and a second test paper strip, wherein the first test paper strip and the second test paper strip respectively comprise: a substrate, and a sample pad, a colloidal gold adsorption pad, an antibody carrying film and a water absorption pad sequentially adhered to the substrate; wherein the sample pad, the colloidal gold adsorption pad, the antibody carrying film, and the water absorption pad are only in contact with and only partially overlap with adjacent pad thereof; the antibody carrying film has a quality control line and a detection line arranged at an interval, the quality control line is close to the water absorption pad, and the detection line is close to the colloidal gold adsorption pad.

Preferably, colloidal gold-anti-human chorionic gonadotropin β-hCG monoclonal antibody conjugate is adsorbed in the colloidal gold adsorption pad of the first test paper strip, and colloidal gold-anti-human chorionic gonadotropin β-hCG monoclonal antibody conjugate and a free β antibody are absorbed in the colloidal gold adsorption pad of the second test paper strip.

Preferably, the detection line is coated with an anti-human chorionic gonadotropin α-hCG monoclonal antibody; the quality control line is coated with an anti-mouse IgG polyclonal antibody.

The free β antibody is a free human chorionic gonadotropin β-hCG antibody.

Preferably, the antibody carrying film is a nitrocellulose film having a pore size of 3-10 um.

Preferably, the sample pad and the water absorption pad are each provided with a protective film.

The present application also provides a method for preparing a hCG cycle test paper strip, comprising the steps of:
S10: coating an antibody carrying film with a detection line and a quality control line disposed at an interval of 0.3-0.7cm in parallel, and drying at a constant temperature of 4-35 °C;
S20: immersing the antibody carrying film obtained in step S10 in PBS buffer solution, and then immersing in a blocking treatment solution, and drying at a temperature of 18-28 °C and a relative humidity of ≤40%;
S30: preparing a colloidal gold complex solution and adjusting pH to 6.5-7.0, adding anti-human chorionic gonadotropin β-hCG monoclonal antibody, and then adding a stabilizer to mix well, centrifuging and collecting a precipitate, using the colloidal gold complex solution to reconstitute the precipitate, casting on a colloidal gold adsorption pad of a first test paper strip, and followed by drying, sealing and storing;
S40: preparing a colloidal gold complex solution and adjusting pH to 6.5-7.0, adding anti-human chorionic gonadotropin β-hCG monoclonal antibody, and then adding a stabilizer to mix well, centrifuging and collecting a precipitate, using the colloidal gold complex solution to reconstitute the precipitate, and then adding free β antibody to mix well, casting on a colloidal gold adsorption pad of a second test paper strip, and followed by drying, sealing and storing;
S50: adhering a sample pad, the colloidal gold adsorption pad of the first test paper strip, the antibody carrying film, and a water adsorption pad sequentially along a length direction of a substrate to obtain a first test paper strip; and adhering the colloidal gold adsorption pad of the second test paper strip, the antibody carrying film, and a water adsorption pad sequentially along a length direction of a substrate to obtain a second test paper strip.

Preferably, a concentration of anti-human chorionic gonadotropinα-hCG monoclonal antibody used for coating the detection line is 1.1-1.5 mg/mL, preferably 1.3 mg/mL; a concentration of anti-mouse IgG polyclonal antibody used for coating the quality control line is 1.5-2.5 mg/mL, preferably 2 mg/mL.

Preferably, the blocking treatment solution comprises 0.08-0.12Mol buffer solution, 0.3-0.7wt% sugar, and 0.8-1.2wt% blocking protein, and 0.03-0.08wt% preservative, preferably 0.1Mol buffer solution, 0.5wt% sugar, 1wt% blocking protein, and 0.05wt% preservative.

Preferably, the buffer solution is phosphate buffer, the sugar is sucrose or trehalose, the preservative is NaN₃ or thimerosal, and the blocking protein is casein or bovine serum albumin.

Preferably, a concentration of the anti-human chorionic gonadotropin β-hCG monoclonal antibody in the step S30 is 5 µg/mL.

Preferably, a concentration of the anti-human chorionic gonadotropin β-hCG monoclonal antibody in the step S40 is 5 µg/mL, and a weight ratio of the free β antibody to the anti-human chorionic gonadotropin β-hCG monoclonal antibody is 10: 1.

Preferably, the colloidal gold-anti-human chorionic gonadotropin β-hCG monoclonal antibody conjugate is cast on the colloidal gold adsorption pad of the first test paper strip with a casting amount of 50 µl/cm².

Preferably, the colloidal gold-anti-human chorionic gonadotropin β-hCG monoclonal antibody conjugate and the free β antibody are cast on the colloidal gold adsorption pad of the second test paper strip with a casting amount of 50 µl/cm². The present application also provides a hCG cycle test kit, comprising the hCG cycle test paper strip or the hCG cycle test paper strip prepared by the method.

Preferably, the hCG cycle test kit further comprises a casing, wherein the casing is provided with two grooves arranged in parallel and used for placing the first test paper strip and the second test paper strip, respectively.

Preferably, the hCG cycle test kit further comprises a cover body, wherein the cover body is provided with two parallel windows and detachably covered on the casing, and the detection line and quality control line of the first test paper strip and the second test paper strip can be observed through the windows.

The present application also provides use of the hCG cycle test paper strip or the hCG cycle test paper strip prepared by the method or the hCG cycle test kit in detecting human pregnancy time.

The present application also provides a method for detecting human pregnancy time by using the hCG cycle test paper strip, comprising the steps of:
a) taking urine of a person to be tested, and then completely soaking sample pads of the first test paper strip and the second test paper strip into the urine for 3-10 seconds, and then taking the sample pads out and laying flat, observing a result within 3-5 minutes, the result is invalid after 5 minutes;
b) observing color depth of detection lines T1 and T2 of the first test paper strip and the second test paper strip and whether quality control line C1 of the first test paper strip and quality control line C2 of the second test paper strip are colored or not:
   if quality control lines C1 and C2 develop color, and detection lines T1 and T2 do not develop color, it indicates that the tested person is not pregnant;
   if quality control lines C1 and C2 develop color, and detection line T1 develops color, and detection line T2 does not develop color, it indicates that the tested person is pregnant and has a pregnancy time of 1-2 weeks;
   if quality control lines C1 and C2 develop color, detection line T1 develops color, detection line T2 develops lighter color than detection line T1, it indicates that the tested person is pregnant and has a pregnancy time of 2-3 weeks;
   if quality control lines C1 and C2 develop color, detection line T1 develops color, detection line T2 develops lighter or closer to detection line T1, it indicates that the tested person is pregnant and has a pregnancy time of more than 3 weeks; and
   if neither quality control lines C1 and C2 nor detection lines T1 and T2 show prunosus, or no quality control lines C1 and C2 show prunosus, it indicates that the hCG cycle test paper strip is invalid.

The technical solutions provided by the present application have the following advantages, compared with the prior art.
(1) The hCG cycle test paper strip provided in the present application comprises a first test paper strip and a second test paper strip, wherein the first test paper strip and the second test paper strip respectively comprise: a substrate, and a sample pad, a colloidal gold adsorption pad, an antibody carrying film and a water absorption pad sequentially adhered to the substrate; wherein the sample pad, the colloidal gold adsorption pad, the antibody carrying film, and the water absorption pad are only in contact with and only partially overlap with adjacent pad thereof; the antibody carrying film has a quality control line and a detection line arranged at an interval, the quality control line is close to the water absorption pad, and the detection line is close to the colloidal gold adsorption pad; colloidal gold-anti-human chorionic gonadotropin β-hCG monoclonal antibody conjugate is adsorbed in the colloidal gold adsorption pad of the first test paper strip, and colloidal gold-anti-human chorionic gonadotropin β-hCG monoclonal antibody conjugate and a free β antibody are absorbed in the colloidal gold adsorption pad of the second test paper strip; the detection line is coated with an anti-human chorionic gonadotropin α-hCG monoclonal antibody; the quality control line is coated with an anti-mouse IgG polyclonal antibody. The above hCG cycle test paper strip only comprises two test paper strips, and can be used for a urine sample which does not need to be processed, the operation and detection method are simple, thereby having good specificity, repeatability, stability and sensitivity. Since the minimum detectable quantity of the first test paper strip is not higher than 25 mIU/ml, and the minimum detectable quantity of the second test paper strip is not higher than 100 mIU/ml, the above hCG cycle test paper strip can accurately detect the number of days of pregnancy, and the detection process is only 5min, and thus it is suitable for on-site, instant, and rapid detection and also suitable for the detection of the number of days of pregnancy in a family.
(2) hCG cycle test kit provided in the present application can be used to qualitatively detect the number of days of pregnancy by a simple method, suitable for on-site, instant, and rapid detection. Compared with the existing test paper strip, it has obvious advantages in terms of detection speed, portability and field applicability, and can accurately detect the number of days of pregnancy.
(3) The method for detecting the number of days of pregnancy by using hCG cycle test paper strip or hCG cycle test kit provided in the present application is simple and easy operation, and can be used to qualitatively detect the number of days of pregnancy, and thereby being suitable for on-site, instant, and rapid detection. Compared with the existing test paper strip, it has obvious advantages in terms of detection speed, portability and field applicability.

### DESCRIPTION OF THE DRAWING

Fig. 1 is a schematic view showing a first test paper strip of a hCG cycle test paper strip according to the present application;
Fig.2 is a schematic view showing a second test paper strip of a hCG cycle test paper strip according to the present application;
Fig. 3 (a) is a schematic view showing a hCG cycle detection kit according to Example 5 of the present application;
Fig. 3 (b) is a schematic view showing a hCG cycle detection kit according to Example 5 of the present application;
Fig. 4 is a schematic view of using a hCG cycle detection kit according to Example 9 of the present application;
Fig. 5 is a schematic view showing a hCG cycle detection kit according to Example 7 of the present application;
Fig. 6 is a schematic view showing a hCG cycle detection kit according to Example 9 of the present application;
Fig. 7 is a schematic view showing a hCG cycle detection kit according to Example 8 of the present application.

In the figures, the reference numerals are: 1-first test paper strip, 2-second test paper strip, 3-cover body, 4-casing, 11-first plastic substrate, 12-first sample pad, 13-first water adsorption pad, 14-first protective film, 15-first nitrocellulose film, 16-first colloidal gold adsorption pad, T1-first detection line, C1-first quality control line, 21-second plastic substrate, 22-second sample pad, 23 -second water adsorption pad, 24-second protective film, 25-second nitrocellulose film, 26-second colloidal gold adsorption pad, T2-second detection line, C2-second quality control line.

### DETAILED DESCRIPTION OF THE EMBODIMENTS

In order to make the objectives, technical solutions, and advantages of the present application clearer, the following specific examples are used to explain the embodiments of the present application. The experimental methods disclosed in the present application adopt conventional techniques in the art, unless otherwise stated.

### Example 1

Example 1 provides a hCG cycle test paper strip, comprising a first test paper strip 1 and a second test paper strip 2. The first test paper strip 1 and the second test paper strip respectively 2 comprise: a substrate, and a sample pad, a colloidal gold adsorption pad, an antibody carrying film and a water absorption pad sequentially adhered to the substrate. The sample pad, the colloidal gold adsorption pad, the antibody carrying film, and the water absorption pad are only in contact with and only partially overlap with adjacent pad thereof. The antibody carrying film is adhered to a middle part of the substrate, and has a quality control line and a detection line arranged at an interval. The quality control line is close to the water absorption pad, and the detection line is close to the colloidal gold adsorption pad. Colloidal gold-anti-human chorionic gonadotropin β-hCG monoclonal antibody conjugate is adsorbed in the colloidal gold adsorption pad of the first test paper strip, and colloidal gold-anti-human chorionic gonadotropin β-hCG monoclonal antibody conjugate and a free β antibody are absorbed in the colloidal gold adsorption pad of the second test paper strip. The detection line is coated with an anti-human chorionic gonadotropin α-hCG monoclonal antibody; the quality control line is coated with an anti-mouse IgG polyclonal antibody.

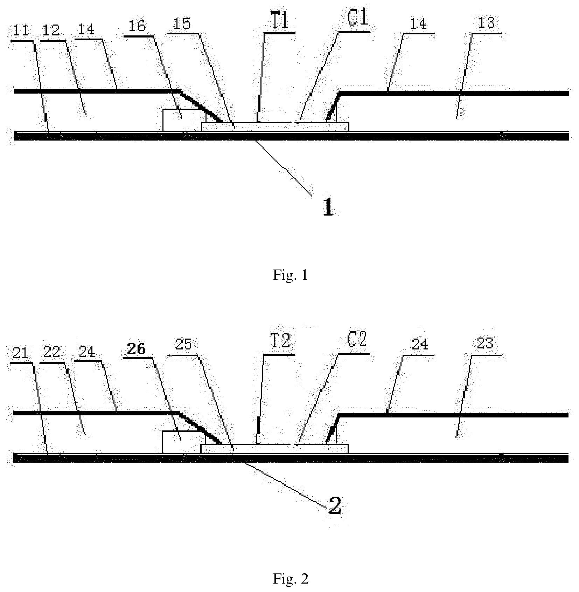

In example 1, as shown in Fig. 1, the first test paper strip 1 comprises a first plastic substrate 11, and a first sample pad 12, a first colloidal gold adsorption pad 16, a first antibody carrying film and a first water adsorption pad 13 sequentially adhered to the first plastic substrate 11. The first antibody carrying film is a first nitrocellulose film 15 located between and below the first sample pad 12 and the first water adsorption pad 13. The first nitrocellulose film 15 is lapped with the first water adsorption pad 13, one end of the first sample pad 12 is lapped with the first colloidal gold adsorption pad 16, and the first colloidal gold adsorption pad 16 is lapped with the first nitrocellulose film 15. The first nitrocellulose film 15 is coated with a first detection line T1 and a first quality control line C1 arranged at an interval, the detection line T1 is close to the first sample pad 12, the first quality control line C1 is close to the first water adsorption pad 13. Colloidal gold-anti-human chorionic gonadotropin β-hCG monoclonal antibody conjugate is adsorbed in the first colloidal gold adsorption pad 16. The first detection line T1 is coated with an anti-human chorionic gonadotropin α-hCG monoclonal antibody; the first quality control line C1 is coated with an anti-mouse IgG polyclonal antibody.

As shown in Fig. 2, the second test paper strip 2 comprises a second plastic substrate 21, and a second sample pad 22, a second colloidal gold adsorption pad 26, a second antibody carrying film and a second water adsorption pad 23 sequentially adhered to the second plastic substrate 21. The second antibody carrying film is a second nitrocellulose film 25 located between and below the second sample pad 22 and the second water adsorption pad 23. The second nitrocellulose film 25 is lapped with the second water adsorption pad 23, one end of the second sample pad 22 is lapped with the second colloidal gold adsorption pad 26, and the second colloidal gold adsorption pad 26 is lapped with the second nitrocellulose film 25. The second nitrocellulose film 25 is coated with a second detection line T2 and a second quality control line C2 arranged at an interval, the detection line T2 is close to the second sample pad 22, the second quality control line C2 is close to the second water adsorption pad 23. The second colloidal gold adsorption pad 26 is adsorbed with colloidal gold-anti-human chorionic gonadotropin β-HCG monoclonal antibody conjugate and a free β antibody. The second detection line T2 is coated with an anti-human chorionic gonadotropinα-HCG monoclonal antibody; the second quality control line C2 is coated with an anti-mouse IgG polyclonal antibody.

Moreover, the first sample pad 12 and the first water absorption pad 13 are each provided with a first protective film 14, and the second sample pad 22 and the second water absorption pad 23 are each provided with a second protective film 24.

Moreover, the first quality control line C1 and the first detection line T1 are arranged in parallel with each other and at an interval of 0.3-0.7 cm. In this example, the first quality control line C1 and the first detection line T1 are arranged at an interval of 0.5cm. The second quality control line C2 and the second detection line T2 are arranged in parallel with each other and at an interval of 0.3-0.7 cm. In this example, the second quality control line C2 is 0.5 cm from the second detection line T2.

Moreover, the first nitrocellulose film 15 and the second nitrocellulose film 25 are a nitrocellulose film having a pore size of 3-10 um.

### Example 2

Example 2 provides a method for preparing a hCG cycle test paper strip of example 1, comprising the following steps:
1. Preparation of a nitrocellulose film:
   (1) A nitrocellulose film with a pore size of 3-10 um is cut into a film having specifications with a width greater than or equal to 2.0 cm and a length of 30.5cm, as needed.
   (2) 1.3mg/ml anti-α-HCG monoclonal antibody is prepared with 0.1M phosphate buffer for coating of the detection line, and 2.0 mg/ml anti-mouse IgG polyclonal antibody is prepared with 0.85 wt% sodium chloride buffer for coating of the quality control line.
   (3) An antibody coating surface of the nitrocellulose film is labeled. The antibody solution of the detection line to be coated and the antibody solution of the quality control line to be coated should be uniformly coated on the film in parallel, and the detection line and the quality control line should be coated. The detection line and the quality control line are disposed at an interval of 0.5cm. The nitrocellulose film is dried at a constant temperature of 4 -35 ° C.
   (4) A blocking treatment soaking solution is prepared. An actual production volume of purified water is added to a mixing tank; and then 0.1Mol phosphate buffer, 0.5wt% sugar, and 1wt% blocking protein and 0.05wt% preservative are weighed with an electronic analytical balance and then directly added to a mixing tank with stirring until completely dissolved, purified water is added to reach a required volume, followed by stirring well for not less than 10 minutes.
   (5) The coating film is placed in a processing tank, and the prepared 0.01M PBS buffer at a temperature of about 36 ° C is added therein. It should be ensured that each film is completely immersed in the 0.01M PBS buffer for 5 minutes (the film does not move and overlap). The film is taken from the processing tank, and then the 0.01M PBS buffer is discard. Then the film immersed once with the 0.01M PBS buffer for 5 minutes is placed into the processing tank, then the prepared 0.01M PBS buffer is added so as to ensure that each film is completely immersed in the 0.01M PBS buffer solution for 30 minutes (the film does not move or overlap). After that, the film is taken from the processing tank and 0.01M PBS buffer solution is discarded. Then the film immersed twice with the 0.01M PBS buffer is placed into the processing tank and immersed with the prepared blocking treatment solution so as to ensure that each film is completely immersed in the treatment solution for 15 minutes (the film does not move or overlap). After that, the film is taken from the processing tank, and the film of each frame is pulled aside with tweezers to ensure that the film does not move outside the frame and does not overlap, and followed by immersing the frame and the film in the treatment solution for 20 minutes again. After that, the film is taken from the processing tank and then pulled on a gauze with tweezers to dry it a little (pay attention to the action of drying the film, do not break or deform the film, pay attention to the degree of drying of the film).
   (6) Pasted on a board and drying

   A white paper in the middle of a cutting line on a double-sided tape of a tape board is removed. An operator should wear disposable gloves in the left hand and hold tweezers in the right hand, and place the film in the blank space in the center of the tape board, and make sure that a corner cut is located at the upper right side of the board, and the right side of the tape board is flush with the right side of the film. In order to avoid errors in the production process, it is necessary to ensure that the color development position is relatively accurate. The film is pasted on the board by aligning with the top of one end of the quality control line. After the film is pasted on the tape board, the film surface is smoothed across double-sided tape to avoid air bubbles. The temperature in the room is controlled to 18-28 °C, and the relative humidity is ≤40%. It is also necessary to ensure that an air in a drying room could circulate and a wind of a dehumidifier will not directly blow on the film surface. The drying time is ≥4 hours.
2. Preparation of the first colloidal gold adsorption pad
   (1) Preparation of colloidal gold complex solution
      The actual production volume of purified water is added to the mixing tank; and then 5wt% trehalose, 2wt% bovine serum albumin, 0.5wt% trisodium citrate, 0.05wt% polyethylene glycol, and 0.05wt% NaN₃ are weighed with an electronic analytical balance and then directly added to the mixing tank with stirring until completely dissolved, purified water is added to reach a required volume, followed by stirring well for not less than 30 minutes, thus preparing a colloidal gold complex solution.
   (2) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-human chorionic gonadotropin β-hCG monoclonal antibody is labeled with the adjusted colloidal gold at 5 µg/ml, i.e. the anti-human chorionic gonadotropin β-hCG monoclonal antibody is diluted with double-distilled water at 5% by volume with mixing well to obtain anti-human chorionic gonadotropin β-hCG monoclonal antibody solution with a concentration of 5 µg/ml which is added into the adjusted colloidal gold, followed by stirring on a magnetic stirrer for 30 minutes, and then 0.5% by volume of stabilizer is added with stirring for 30 minutes, followed by centrifuging, collecting a labeled colloidal gold precipitate which is re-dissolved with the colloidal gold complex solution at 3% by volume, and stirring on a magnetic stirrer until the mixture is homogeneous, thus obtaining a re-dissolved colloidal gold solution.
   (3) 3% by volume of the re-dissolved colloidal gold solution of the above step (2) is taken and re-dissolved with the colloidal gold complex solution at 40% by volume, followed by mixing on a magnetic stirrer. The uniformly mixed colloidal gold solution is cast on the prepared colloidal gold adsorption pad at 50 µl/cm², followed by placing in a drying room to dry for> 4 hours, and controlling the temperature in the drying room at 18-28 ° C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as a first colloidal gold adsorption pad. Notes: The colloidal gold solution is cast, because the casted colloidal gold can be cut freely, thus it is easy to adjust the color depth of the product.
3. Preparation of the second colloidal gold adsorption pad
   (1) A required amount of colloidal gold is measured with a measuring cylinder, adjusting PH to 6.5-7.0by adding 0.2 mol/L potassium carbonate solution with 0.53% by volume of the colloidal gold with stirring on a magnetic stirrer for 15 minutes, thus obtaining an adjusted colloidal gold. Anti-human chorionic gonadotropin β-hCG monoclonal antibody is labeled with the adjusted colloidal gold at 5 µg/ml, and then is diluted with double-distilled water at 5% by volume with mixing well to obtain anti-human chorionic gonadotropin β-hCG monoclonal antibody solution with a concentration of 5 µg/ml which is added into the adjusted colloidal gold, followed by stirring on a magnetic stirrer for 30 minutes, and then 0.5% by volume of stabilizer is added with stirring for 30 minutes, followed by centrifuging, collecting a precipitate which is re-dissolved with colloidal gold complex solution at 3% by volume, and stirring on a magnetic stirrer until the mixture is homogeneous.
   (2) 3% by volume of the re-dissolved colloidal gold in the above step (1) is taken and re-dissolved with the colloidal gold complex solution at 40% by volume, and then free β antibody is added according to a mass ratio of anti-human chorionic gonadotropin β-hCG monoclonal antibody to free β antibody of 1:10, followed by mixing on a magnetic stirrer. The uniformly mixed colloidal gold solution is caston the prepared colloidal gold adsorption pad at 50 µl/cm², followed by placing in a drying room to dry for ≥ 4 hours, and controlling the temperature in the drying room at 18-28 ° C, and the relative humidity ≤ 40%. It should be ensured that the air is unobstructed and that the air flow cannot be blown directly onto the colloidal gold adsorption pad. The dried labeled gold is placed into an aluminum foil bag containing a desiccant, sealed for storage, and labeled as a second colloidal gold adsorption pad. Notes: The colloidal gold solution is cast, because the casted colloidal gold can be cut freely, thus it is easy to adjust the color depth of the product.
4. Assembly and cutting:
   (1) A transparent substrate semi-finished product that has been pasted with the nitrocellulose film is taken, and immobilized first colloidal gold adsorption pad is cut into 0.5cm × 30cm strip with a width of 0.5cm which is pasted on the transparent substrate and kept to lap about 1mm with the nitrocellulose film. The first water adsorption pad is compounded on the upper end of the transparent substrate film of the nitrocellulose film and lapped about 1mm with the film. The first sample pad is compounded on the lower end of the immobilized first colloidal gold adsorption pad and lapped about 1mm therewith, labeling for use.
   (2) According to the corresponding reaction device, the assembled substrate is cut into a strip test paper labeled as a first test paper strip.
   (3) A transparent substrate semi-finished product that has been pasted with the nitrocellulose film is taken, and immobilized second colloidal gold adsorption pad is cut into 0.5cm × 30cm strip with a width of 0.5cm which is pasted on the transparent substrate and kept to lap about 1mm with the nitrocellulose film. The second water adsorption pad is compounded on the upper end of the transparent substrate film of the nitrocellulose film and lapped about 1mm with the film. The second sample pad is compounded on the lower end of the immobilized second colloidal gold adsorption pad and lapped about 1mm therewith, labeling for use.
   (4) According to the corresponding reaction device, the assembled substrate is cut into a strip test paper labeled as a second test paper strip.

### Example 3

Example 3 provides a method for preparing a hCG cycle test paper strip of example 1, comprising the following steps:
1. Preparation of a nitrocellulose film:
   (1) A nitrocellulose film with a pore size of 3-0 um is cut into a film having specifications with a width greater than or equal to 2.0 cm and a length of 30.5cm, as needed.
   (2) 1.1mg/ml anti-α-HCG monoclonal antibody is prepared with 0.1M phosphate buffer for coating of the detection line, and 2.5 mg/ml anti-mouse IgG polyclonal antibody is prepared with 0.85 wt% sodium chloride buffer for coating of the quality control line.
   (3) An antibody coating surface of the nitrocellulose film is labeled. The antibody solution of the detection line to be coated and the antibody solution of the quality control line to be coated should be uniformly coated on the film in parallel, and the detection line and the quality control line should be coated. The detection line and the quality control line are disposed at an interval of 0.5cm. The nitrocellulose film is dried at a constant temperature of 4 -35 ° C.
   (4) A blocking treatment soaking solution is prepared. An actual production volume of purified water is added to a mixing tank; and then phosphate buffer, sugar, bovine serum albumin and thimerosal are weighed with an electronic analytical balance and then directly added to the mixing tank with stirring until completely dissolved, purified water is added to reach a required volume, followed by stirring well for not less than 10 minutes. In the prepared blocking treatment soaking solution, the concentration of the phosphate buffer solution is 0.08 Mol, the weight percentage of sugar is 0.7%, the weight percentage of bovine serum albumin is 0.8%, and the weight percentage of thimerosal is 0.08%.
   (5) The coating film is placed in a processing tank, and the prepared 0.01M PBS buffer at a temperature of about 36 ° C is added therein. It should be ensured that each film is completely immersed in the 0.01M PBS buffer for 5 minutes (the film does not move and overlap). The film is taken from the processing tank, and then the 0.01M PBS buffer is discard. Then the film immersed once with the 0.01M PBS buffer for 5 minutes is placed into the processing tank, then the prepared 0.01M PBS buffer is added so as to ensure that each film is completely immersed in the 0.01M PBS buffer solution for 30 minutes (the film does not move or overlap). After that, the film is taken from the processing tank and 0.01M PBS buffer solution is discarded. Then the film immersed twice with the 0.01M PBS buffer is placed into the processing tank and immersed with the prepared blocking treatment solution so as to ensure that each film is completely immersed in the treatment solution for 15 minutes (the film does not move or overlap). After that, the film is taken from the processing tank, and the film of each frame is pulled aside with tweezers to ensure that the film does not move outside the frame and does not overlap, and followed by immersing the frame and the film in the treatment solution for 20 minutes again. After that, the film is taken from the processing tank and then pulled on a gauze with tweezers to dry it a little (pay attention to the action of drying the film, do not break or deform the film, pay attention to the degree of drying of the film).
   (6) Pasted on a board and drying

   A white paper in the middle of a cutting line on a double-sided tape of a tape board is removed. An operator should wear disposable gloves in the left hand and hold tweezers in the right hand, and place the film in the blank space in the center of the tape board, and make sure that a corner cut is located at the upper right side of the board, and the right side of the tape board is flush with the right side of the film. In order to avoid errors in the production process, it is necessary to ensure that the color development position is relatively accurate. The film is pasted on the board by aligning with the top of one end of the quality control line. After the film is pasted on the tape board, the film surface is smoothed across double-sided tape to avoid air bubbles. The temperature in the room is controlled to 18-28 °C, and the relative humidity is ≤40%. It is also necessary to ensure that an air in a drying room could circulate and a wind of a dehumidifier will not directly blow on the film surface. The drying time is ≥4 hours.
2. Preparation of the first colloidal gold adsorption pad
   (1) Preparation of colloidal gold complex solution: The actual production volume of purified water is added to the mixing tank; and then trehalose, bovine serum albumin, trisodium citrate, polyethylene glycol, and NaN₃ are weighed with an electronic analytical balance and then directly added to the mixing tank with stirring until completely dissolved, purified water is added to reach a required volume, followed by stirring well for not less than 30 minutes, thus preparing a colloidal gold complex solution. The prepared colloidal gold complex solution contains 5wt% trehalose, 2wt% bovine serum albumin, 0.5wt% trisodium citrate, and 0.05wt% polyethylene glycol, and 0.05wt% NaN₃.
   (2) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-human chorionic gonadotropin β-hCG monoclonal antibody is labeled with the adjusted colloidal gold at 5 µg/ml, i.e. an appropriate amount of anti-human chorionic gonadotropin β-hCG monoclonal antibody is diluted with double-distilled water at 5% by volume with mixing well to obtain anti-human chorionic gonadotropin β-hCG monoclonal antibody solution with a concentration of 5 µg/ml which is added into the adjusted colloidal gold, followed by stirring on a magnetic stirrer for 30 minutes, and then 0.5% by volume of stabilizer is added with stirring for 30 minutes, followed by centrifuging, collecting a labeled colloidal gold precipitate, which is re-dissolved with the colloidal gold complex solution, and followed by stirring on a magnetic stirrer until the mixture is homogeneous, thus obtaining a re-dissolved colloidal gold solution. The volume percentage of the labeled colloidal gold precipitate in the colloidal gold complex solution is 3%.
   (3) The re-dissolved colloidal gold solution of the above step (2) is taken and re-dissolved with the colloidal gold complex solution at 40% by volume, followed by mixing on a magnetic stirrer. The uniformly mixed colloidal gold solution is cast on the prepared colloidal gold adsorption pad at 50 µl/cm², followed by placing in a drying room to dry for ≥ 4 hours, and controlling the temperature in the drying room at 18-28 ° C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as a first colloidal gold adsorption pad. Notes: The colloidal gold solution is cast, because the casted colloidal gold can be cut freely, thus it is easy to adjust the color depth of the product.
3. Preparation of the second colloidal gold adsorption pad
   (1) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-human chorionic gonadotropin β-hCG monoclonal antibody is labeled with the adjusted colloidal gold at 5 µg/ml, i.e. an appropriate amount of anti-human chorionic gonadotropin β-hCG monoclonal antibody is diluted with double-distilled water at 5% by volume with mixing well to obtain anti-human chorionic gonadotropin β-hCG monoclonal antibody solution with a concentration of 5 µg/ml which is added into the adjusted colloidal gold, followed by stirring on a magnetic stirrer for 30 minutes, and then 0.5% by volume of stabilizer is added with stirring for 30 minutes, followed by centrifuging, collecting a labeled colloidal gold precipitate, which is re-dissolved with the colloidal gold complex solution, and followed by stirring on a magnetic stirrer until the mixture is homogeneous, thus obtaining a re-dissolved colloidal gold solution. The volume percentage of the labeled colloidal gold precipitate in the colloidal gold complex solution is 3%.
   (2) The re-dissolved colloidal gold solution of the above step (1) is taken and re-dissolved with the colloidal gold complex solution at 40% by volume, and then free β antibody is added according to a mass ratio of anti-human chorionic gonadotropin β-hCG monoclonal antibody to free β antibody of 1:10, followed by mixing on a magnetic stirrer. The uniformly mixed colloidal gold solution is cast on the prepared colloidal gold adsorption pad at 50 µl/cm², followed by placing in a drying room to dry for ≥ 4 hours, and controlling the temperature in the drying room at 18-28 ° C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as a second colloidal gold adsorption pad. Notes: The colloidal gold solution is cast, because the casted colloidal gold can be cut freely, thus it is easy to adjust the color depth of the product.
4. Assembly and cutting:
   (1) A transparent substrate semi-finished product that has been pasted with the nitrocellulose film is taken, and immobilized first colloidal gold adsorption pad is cut into 0.5cm × 30cm strip with a width of 0.5cm which is pasted on the transparent substrate and kept to lap about 1mm with the nitrocellulose film. The first water adsorption pad is compounded on the upper end of the transparent substrate film of the nitrocellulose film and lapped about 1mm with the film. The first sample pad is compounded on the lower end of the immobilized first colloidal gold adsorption pad and lapped about 1mm therewith, labeling for use.
   (2) According to the corresponding reaction device, the assembled substrate is cut into a strip test paper labeled as a first test paper strip.
   (3) A transparent substrate semi-finished product that has been pasted with the nitrocellulose film is taken, and immobilized second colloidal gold adsorption pad is cut into 0.5cm × 30cm strip with a width of 0.5cm which is pasted on the transparent substrate and kept to lap about 1mm with the nitrocellulose film. The second water adsorption pad is compounded on the upper end of the transparent substrate film of the nitrocellulose film and lapped about 1mm with the film. The second sample pad is compounded on the lower end of the immobilized second colloidal gold adsorption pad and lapped about 1mm therewith, labeling for use.
   (4) According to the corresponding reaction device, the assembled substrate is cut into a strip test paper labeled as a second test paper strip.

### Example 4

Example 4 provides a method for preparing a hCG cycle test paper strip of example 1, comprising the following steps:
1. Preparation of a nitrocellulose film:
   (1) A nitrocellulose film with a pore size of 3-10 um is cut into a film having specifications with a width greater than or equal to 2.0 cm and a length of 30.5cm, as needed.
   (2) 1.5 mg/ml anti-α-HCG monoclonal antibody is prepared with 0.1M phosphate buffer for coating of the detection line, and 1.5 mg/ml anti-mouse IgG polyclonal antibody is prepared with 0.85 wt% sodium chloride buffer for coating of the quality control line.
   (3) An antibody coating surface of the nitrocellulose film is labeled. The antibody solution of the detection line to be coated and the antibody solution of the quality control line to be coated should be uniformly coated on the film in parallel, and the detection line and the quality control line should be coated. The detection line and the quality control line are disposed at an interval of 0.5cm. The nitrocellulose film is dried at a constant temperature of 4 -35 ° C.
   (4) A blocking treatment soaking solution is prepared. An actual production volume of purified water is added to a mixing tank; and then phosphate buffer, sugar, bovine serum albumin and thimerosal are weighed with an electronic analytical balance and then directly added to the mixing tank with stirring until completely dissolved, purified water is added to reach a required volume, followed by stirring well for not less than 10 minutes. In the prepared blocking treatment soaking solution, the concentration of the phosphate buffer solution is 0.12 Mol, the weight percentage of sugar is 0.3%, the weight percentage of bovine serum albumin is 1.2%, and the weight percentage of thimerosal is 0.03%.
   (5) The coating film is placed in a processing tank, and the prepared 0.01M PBS buffer at a temperature of about 36 ° C is added therein. It should be ensured that each film is completely immersed in the 0.01M PBS buffer for 5 minutes (the film does not move and overlap). The film is taken from the processing tank, and then the 0.01M PBS buffer is discard. Then the film immersed once with the 0.01M PBS buffer for 5 minutes is placed into the processing tank, then the prepared 0.01M PBS buffer is added so as to ensure that each film is completely immersed in the 0.01M PBS buffer solution for 30 minutes (the film does not move or overlap). After that, the film is taken from the processing tank and 0.01M PBS buffer solution is discarded. Then the film immersed twice with the 0.01M PBS buffer is placed into the processing tank and immersed with the prepared blocking treatment solution so as to ensure that each film is completely immersed in the treatment solution for 15 minutes (the film does not move or overlap). After that, the film is taken from the processing tank, and the film of each frame is pulled aside with tweezers to ensure that the film does not move outside the frame and does not overlap, and followed by immersing the frame and the film in the treatment solution for 20 minutes again. After that, the film is taken from the processing tank and then pulled on a gauze with tweezers to dry it a little (pay attention to the action of drying the film, do not break or deform the film, pay attention to the degree of drying of the film).
   (6) Pasted on a board and drying

   A white paper in the middle of a cutting line on a double-sided tape of a tape board is removed. An operator should wear disposable gloves in the left hand and hold tweezers in the right hand, and place the film in the blank space in the center of the tape board, and make sure that a corner cut is located at the upper right side of the board, and the right side of the tape board is flush with the right side of the film. In order to avoid errors in the production process, it is necessary to ensure that the color development position is relatively accurate. The film is pasted on the board by aligning with the top of one end of the quality control line. After the film is pasted on the tape board, the film surface is smoothed across double-sided tape to avoid air bubbles. The temperature in the room is controlled to 18-28 °C, and the relative humidity is ≤40%. It is also necessary to ensure that an air in a drying room could circulate and a wind of a dehumidifier will not directly blow on the film surface. The drying time is ≥4 hours.
2. Preparation of the first colloidal gold adsorption pad
   (1) Preparation of colloidal gold complex solution: The actual production volume of purified water is added to the mixing tank; and then trehalose, bovine serum albumin, trisodium citrate, polyethylene glycol, and NaN₃ are weighed with an electronic analytical balance and then directly added to the mixing tank with stirring until completely dissolved, purified water is added to reach a required volume, followed by stirring well for not less than 30 minutes, thus preparing a colloidal gold complex solution. The prepared colloidal gold complex solution contains 5wt% trehalose, 2wt% bovine serum albumin, 0.5wt% trisodium citrate, and 0.05wt% polyethylene glycol, and 0.05wt% NaN₃.
   (2) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-human chorionic gonadotropin β-hCG monoclonal antibody is labeled with the adjusted colloidal gold at 5 µg/ml, i.e. an appropriate amount of anti-human chorionic gonadotropin β-hCG monoclonal antibody is diluted with double-distilled water at 5% by volume with mixing well to obtain anti-human chorionic gonadotropin β-hCG monoclonal antibody solution with a concentration of 5 µg/ml which is added into the adjusted colloidal gold, followed by stirring on a magnetic stirrer for 30 minutes, and then 0.5% by volume of stabilizer is added with stirring for 30 minutes, followed by centrifuging, collecting a labeled colloidal gold precipitate, which is re-dissolved with the colloidal gold complex solution, and followed by stirring on a magnetic stirrer until the mixture is homogeneous, thus obtaining a re-dissolved colloidal gold solution. The volume percentage of the labeled colloidal gold precipitate in the colloidal gold complex solution is 3%.
   (3) The re-dissolved colloidal gold solution of the above step (2) is taken and re-dissolved with the colloidal gold complex solution at 40% by volume, followed by mixing on a magnetic stirrer. The uniformly mixed colloidal gold solution is cast on the prepared colloidal gold adsorption pad at 50 µl/cm², followed by placing in a drying room to dry for ≥ 4 hours, and controlling the temperature in the drying room at 18-28 ° C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as a as the first colloidal gold adsorption pad. Notes: The colloidal gold solution is cast, because the casted colloidal gold can be cut freely, thus it is easy to adjust the color depth of the product.
3. Preparation of the second colloidal gold adsorption pad
   (1) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-human chorionic gonadotropin β-hCG monoclonal antibody is labeled with the adjusted colloidal gold at 5 µg/ml, i.e. an appropriate amount of anti-human chorionic gonadotropin β-hCG monoclonal antibody is diluted with double-distilled water at 5% by volume with mixing well to obtain anti-human chorionic gonadotropin β-hCG monoclonal antibody solution with a concentration of 5 µg/ml which is added into the adjusted colloidal gold, followed by stirring on a magnetic stirrer for 30 minutes, and then 0.5% by volume of stabilizer is added with stirring for 30 minutes, followed by centrifuging, collecting a labeled colloidal gold precipitate, which is re-dissolved with the colloidal gold complex solution, and followed by stirring on a magnetic stirrer until the mixture is homogeneous, thus obtaining a re-dissolved colloidal gold solution. The volume percentage of the labeled colloidal gold precipitate in the colloidal gold complex solution is 3%.
   (2) The re-dissolved colloidal gold solution of the above step (1) is taken and re-dissolved with the colloidal gold complex solution at 40% by volume, and then free β antibody is added according to a mass ratio of anti-human chorionic gonadotropin β-hCG monoclonal antibody to free β antibody of 1:10, followed by mixing on a magnetic stirrer. The uniformly mixed colloidal gold solution is cast on the prepared colloidal gold adsorption pad at 50 µl/cm², followed by placing in a drying room to dry for ≥ 4 hours, and controlling the temperature in the drying room at 18-28 ° C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as a second colloidal gold adsorption pad. Notes: The colloidal gold solution is cast, because the casted colloidal gold can be cut freely, thus it is easy to adjust the color depth of the product.
4. Assembly and cutting:
   (1) A transparent substrate semi-finished product that has been pasted with the nitrocellulose film is taken, and immobilized first colloidal gold adsorption pad is cut into 0.5cm × 30cm strip with a width of 0.5cm which is pasted on the transparent substrate and kept to lap about 1mm with the nitrocellulose film. The first water adsorption pad is compounded on the upper end of the transparent substrate film of the nitrocellulose film and lapped about 1mm with the film. The first sample pad is compounded on the lower end of the immobilized first colloidal gold adsorption pad and lapped about 1mm therewith, labeling for use.
   (2) According to the corresponding reaction device, the assembled substrate is cut into a strip test paper labeled as the first test paper strip.
   (3) A transparent substrate semi-finished product that has been pasted with the nitrocellulose film is taken, and immobilized second colloidal gold adsorption pad is cut into 0.5cm × 30cm strip with a width of 0.5cm, which is pasted on the transparent substrate and kept to lap about 1mm with the nitrocellulose film. The second water adsorption pad is compounded on the upper end of the transparent substrate film of the nitrocellulose film and lapped about 1mm with the film. The second sample pad is compounded on the lower end of the immobilized second colloidal gold adsorption pad and lapped about 1mm therewith, labeling for use.
   (4) According to the corresponding reaction device, the assembled substrate is cut into a strip test paper labeled as the second test paper strip.

### Example 5

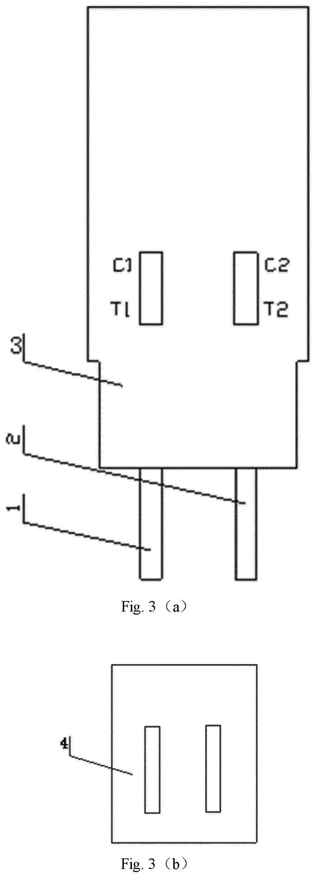

Example 5 provides a hCG cycle test kit, as shown in Fig. 3 (a), includes a first test paper strip 1 and a second test paper strip 2. As shown in Fig. 1, the first test paper strip 1 comprises a first plastic substrate 11, and a first sample pad 12, a first colloidal gold adsorption pad 16, a first nitrocellulose film 15 and a first water adsorption pad 13 sequentially lapped and adhered to each other and then immobilized on the first plastic substrate 11. The first nitrocellulose film 15 is sequentially coated with a first detection line T1 and a first quality control line C1 arranged in parallel, and the first detection line T1 is provided on one side close to the first colloidal adsorption pad 16. The first detection line T1 and the first quality control line C1 are disposed at an interval of 0.5cm. The first colloidal gold adsorption pad 16 is coated with an anti-human chorionic gonadotropin β-hCG monoclonal antibody with a coating concentration of 5 µg/Ml, the first detection line T1 is coated with anti-α-HCG monoclonal antibody, and the first quality control line C1 is coated with anti-mouse IgG polyclonal antibody.

As shown in Fig. 2, the second test paper strip 2 comprises a second plastic substrate 21, and a second sample pad 22, a second colloidal gold adsorption pad 26, a second nitrocellulose film 25 and a second water adsorption pad 23 sequentially lapped and adhered to each other and then immobilized on the second plastic substrate 21. The second nitrocellulose film 25 is sequentially coated with a second detection line T2 and a second quality control line C2 arranged in parallel, and the second detection line T2 is provided on one side close to the second colloidal adsorption pad 26. The second detection line T2 and the second quality control line C2 are disposed at an interval of 0.5cm. The second colloidal gold adsorption pad 26 is coated with an anti-human chorionic gonadotropin β-hCG monoclonal antibody and a free β antibody, the second detection line T2 is coated with anti-α-HCG monoclonal antibody, and the second quality control line C2 is coated with anti-mouse IgG polyclonal antibody;

As shown in Figs. 3 (a) and 3 (b), the hCG cycle test kit also comprises a cover body 3 and a casing 4. The casing 4 is provided with two grooves arranged in parallel, one is installed a first test paper strip 1 therein, the first sample pad 12 extends outside of the casing 4, and the other is installed with a second test paper strip 2 therein, and the second sample pad 22 extends outside of the casing 4. The cover body 3 is provided with two parallel windows and covered on the casing 4. The first detection line T1 and the first quality control line C1 can be observed through the left window on cover body 3, and the second detection line T2 and the second quality control line C2 can be observed through the right window on cover body 3,

### Example 6

Example 6 provides a method for preparing a hCG cycle test kit, comprising the following steps:
1. Preparation of a nitrocellulose film:
   (1) A nitrocellulose film with a pore size of 3-10 um is cut into a film having specifications with a width greater than or equal to 2.0 cm and a length of 30.5cm, as needed.
   (2) 1.3mg/ml anti-α-HCG monoclonal antibody is prepared with 0.1M phosphate buffer for coating of the detection line, and 2.0 mg/ml anti-mouse IgG polyclonal antibody is prepared with 0.85 wt% sodium chloride buffer for coating of the quality control line.
   (3) An antibody coating surface of the nitrocellulose film is labeled. The antibody solution of the detection line to be coated and the antibody solution of the quality control line to be coated should be uniformly coated on the film in parallel, and the detection line and the quality control line should be coated. The detection line and the quality control line are disposed at an interval of 0.5cm. The nitrocellulose film is dried at a constant temperature of 4 -35 ° C.
   (4) A blocking treatment soaking solution is prepared. An actual production volume of purified water is added to a mixing tank; and then phosphate buffer, sugar, bovine serum albumin and thimerosal are weighed with an electronic analytical balance and then directly added to the mixing tank with stirring until completely dissolved, purified water is added to reach a required volume, followed by stirring well for not less than 10 minutes. In the prepared blocking treatment soaking solution, the concentration of the phosphate buffer solution is 0.1 Mol, the weight percentage of sugar is 0.5%, the weight percentage of bovine serum albumin is 1%, and the weight percentage of NaN₃ is 0.05%.
   (5) The coating film is placed in a processing tank, and the prepared 0.01M PBS buffer at a temperature of about 36 ° C is added therein. It should be ensured that each film is completely immersed in the 0.01M PBS buffer for 5 minutes (the film does not move and overlap). The film is taken from the processing tank, and then the 0.01M PBS buffer is discard. Then the film immersed once with the 0.01M PBS buffer for 5 minutes is placed into the processing tank, then the prepared 0.01M PBS buffer is added so as to ensure that each film is completely immersed in the 0.01M PBS buffer solution for 30 minutes (the film does not move or overlap). After that, the film is taken from the processing tank and 0.01M PBS buffer solution is discarded. Then the film immersed twice with the 0.01M PBS buffer is placed into the processing tank and immersed with the prepared blocking treatment solution so as to ensure that each film is completely immersed in the treatment solution for 15 minutes (the film does not move or overlap). After that, the film is taken from the processing tank, and the film of each frame is pulled aside with tweezers to ensure that the film does not move outside the frame and does not overlap, and followed by immersing the frame and the film in the treatment solution for 20 minutes again. After that, the film is taken from the processing tank and then pulled on a gauze with tweezers to dry it a little (pay attention to the action of drying the film, do not break or deform the film, pay attention to the degree of drying of the film).
   (6) Pasted on a board and drying

   A white paper in the middle of a cutting line on a double-sided tape of a tape board is removed. An operator should wear disposable gloves in the left hand and hold tweezers in the right hand, and place the film in the blank space in the center of the tape board, and make sure that a corner cut is located at the upper right side of the board, and the right side of the tape board is flush with the right side of the film. In order to avoid errors in the production process, it is necessary to ensure that the color development position is relatively accurate. The film is pasted on the board by aligning with the top of one end of the quality control line. After the film is pasted on the tape board, the film surface is smoothed across double-sided tape to avoid air bubbles. The temperature in the room is controlled to 18-28 °C, and the relative humidity is ≤40%. It is also necessary to ensure that an air in a drying room could circulate and a wind of a dehumidifier will not directly blow on the film surface. The drying time is ≥4 hours.
2. Preparation of the first colloidal gold adsorption pad
   (1) Preparation of colloidal gold complex solution: The actual production volume of purified water is added to the mixing tank; and then trehalose, bovine serum albumin, trisodium citrate, polyethylene glycol, and NaN₃ are weighed with an electronic analytical balance and then directly added to the mixing tank with stirring until completely dissolved, purified water is added to reach a required volume, followed by stirring well for not less than 30 minutes, thus preparing a colloidal gold complex solution. The prepared colloidal gold complex solution contains 5wt% trehalose, 2wt% bovine serum albumin, 0.5wt% trisodium citrate, and 0.05wt% polyethylene glycol, and 0.05wt% NaN₃.
   (2) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes thus obtaining an adjusted colloidal gold. Anti-human chorionic gonadotropin β-hCG monoclonal antibody is labeled with the adjusted colloidal gold at 5 µg/ml, i.e. an appropriate amount of anti-human chorionic gonadotropin β-hCG monoclonal antibody is diluted with double-distilled water at 5% by volume with mixing well to obtain anti-human chorionic gonadotropin β-hCG monoclonal antibody solution with a concentration of 5 µg/ml which is added into the adjusted colloidal gold, followed by stirring on a magnetic stirrer for 30 minutes, and then 0.5% by volume of stabilizer is added with stirring for 30 minutes, followed by centrifuging, collecting a labeled colloidal gold precipitate, which is re-dissolved with the colloidal gold complex solution, and followed by stirring on a magnetic stirrer until the mixture is homogeneous, thus obtaining a re-dissolved colloidal gold solution. The volume percentage of the labeled colloidal gold precipitate in the colloidal gold complex solution is 3%.
   (3) The re-dissolved colloidal gold solution of the above step (2) is taken and re-dissolved with the colloidal gold complex solution at 40% by volume, followed by mixing on a magnetic stirrer. The uniformly mixed colloidal gold solution is cast on the prepared colloidal gold adsorption pad at 50 µl/cm², followed by placing in a drying room to dry for ≥ 4 hours, and controlling the temperature in the drying room at 18-28 ° C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as a first colloidal gold adsorption pad. Notes: The colloidal gold solution is cast, because the casted colloidal gold can be cut freely, thus it is easy to adjust the color depth of the product.
3. Preparation of the second colloidal gold adsorption pad
   (1) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-human chorionic gonadotropin β-hCG monoclonal antibody is labeled with the adjusted colloidal gold at 5 µg/ml, i.e. an appropriate amount of anti-human chorionic gonadotropin β-hCG monoclonal antibody is diluted with double-distilled water at 5% by volume with mixing well to obtain anti-human chorionic gonadotropin β-hCG monoclonal antibody solution with a concentration of 5 µg/ml which is added into the adjusted colloidal gold, followed by stirring on a magnetic stirrer for 30 minutes, and then 0.5% by volume of stabilizer is added with stirring for 30 minutes, followed by centrifuging, collecting a labeled colloidal gold precipitate, which is re-dissolved with the colloidal gold complex solution, and followed by stirring on a magnetic stirrer until the mixture is homogeneous, thus obtaining a re-dissolved colloidal gold solution. The volume percentage of the labeled colloidal gold precipitate in the colloidal gold complex solution is 3%.
   (2) The re-dissolved colloidal gold solution of the above step (1) is taken and re-dissolved with the colloidal gold complex solution at 40% by volume, and then free β antibody is added according to mass ratio of anti-human chorionic gonadotropin β-hCG monoclonal antibody to free β antibody of 1:10, followed by mixing on a magnetic stirrer. The uniformly mixed colloidal gold solution is cast on the prepared colloidal gold adsorption pad at 50 µl/cm², followed by placing in a dry for ≥ 4 hours, and controlling the temperature in the drying room at 18-28 ° C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as a second colloidal gold adsorption pad. Notes: The colloidal gold solution is cast, because the casted colloidal gold can be cut freely, thus it is easy to adjust the color depth of the product.
4. Assembly and cutting:
   (1) A transparent substrate semi-finished product that has been pasted with the nitrocellulose film is taken, and immobilized first colloidal gold adsorption pad is cut into 0.5cm × 30cm strip with a width of 0.5cm which is pasted on the transparent substrate and kept to lap about 1mm with the nitrocellulose film. The first water adsorption pad is compounded on the upper end of the transparent substrate film of the nitrocellulose film and lapped about 1mm with the film. The first sample pad is compounded on the lower end of the immobilized first colloidal gold adsorption pad and lapped about 1mm therewith, labeling for use.
   (2) According to the corresponding reaction device, the assembled substrate is cut into a strip test paper labeled as a first test paper strip.
   (3) A transparent substrate semi-finished product that has been pasted with the nitrocellulose film is taken, and immobilized second colloidal gold adsorption pad is cut into 0.5cm × 30cm strip with a width of 0.5cm, which is pasted on the transparent substrate and kept to lap about 1mm with the nitrocellulose film. The second water adsorption pad is compounded on the upper end of the transparent substrate film of the nitrocellulose film and lapped about 1mm with the film. The second sample pad is compounded on the lower end of the immobilized second colloidal gold adsorption pad and lapped about 1mm therewith, labeling for use.
   (4) According to the corresponding reaction device, the assembled substrate is cut into a strip test paper labeled as a second test paper strip.
   (5) The qualified first test paper strip 1 is placed into the left groove of the qualified casing 4. The handle paper is arranged above. And then the qualified second test paper strip 2 is placed into the right groove of the casing 4. The cover body 3 is closed tightly. The text on the cover body 3 should correspond to the test strip.

### Example 7

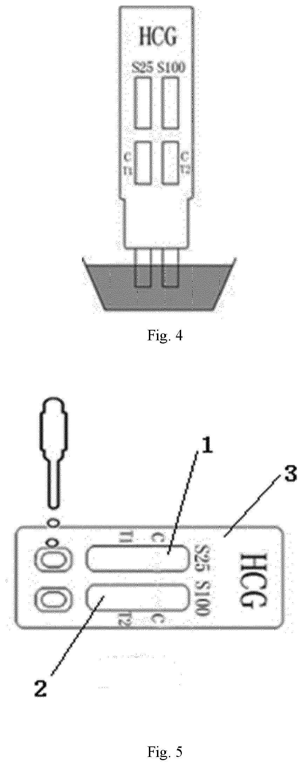

Example 7 provides a hCG cycle test kit, which is different from that of Example 5 in that, as shown in Fig. 5, the first sample pad 12 and the second sample pad 22 do not extend outside the casing 4. The cover body 3 is provided with four windows, two of which correspond to the grooves arranged on casing 4, so that the first detection line T1, the first quality control line C1, the second detection line T2, and the second quality control line C2 are exposed on the cover body 3. The two windows are corresponding to the first sample pad 12 and the second sample pad 22, so that the first sample pad 12 and the second sample pad 22 are exposed on the cover body 3. The sample is dropped on the first sample pad 12 and the second sample pad 22 using a dropper.

### Example 8

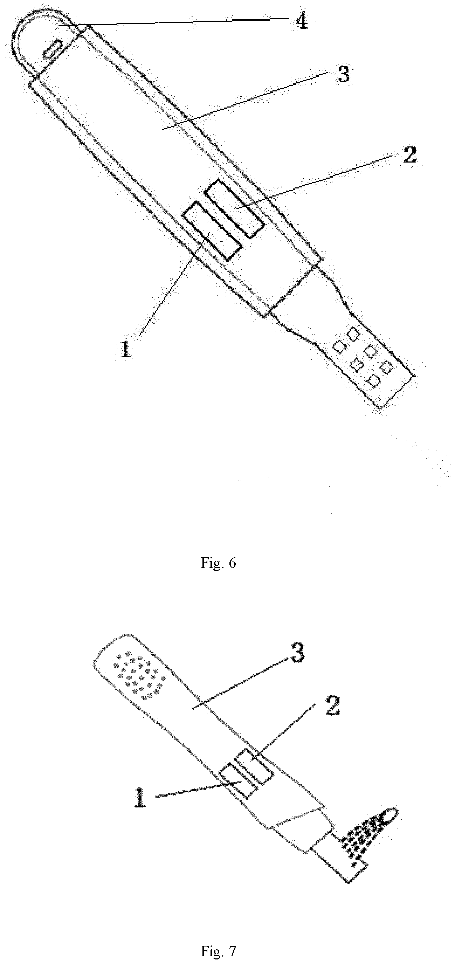

Example 8 provides a hCG cycle test kit, which is different from that of Example 5 or 3 in that, as shown in Fig. 7, the casing 4 is a long pen shape. The first test paper strip 1 and the second test paper strip 2 are installed in the casing 4, the first sample pad 12 and the second sample pad 22 extend outside the casing 4, and the cover body 3 is provided with two windows for observing the detection line and the quality control line.

### Example 9

Example 9 provides a hCG cycle test kit, which is different from that of Example 5, 3 or 8 in that, as shown in Fig. 6, the casing 4 is a long pen shape. The first test paper strip 1 and the second test paper strip 2 are installed in the casing 4. The casing 4 is provided with a protrusion extending in the axial direction in the middle thereof for separating the first test paper strip 1 from the second test paper strip 2, and the casing 4 is also provided with a protruding structure on the top thereof for placing the first sample pad 12 and the second sample pad 22. The cover body 3 is provided with two windows thereon for observing the detection line and the quality control line. The cover body 3 is provided with two rows of parallel windows corresponding to the first sample pad 12 and the second sample pad 22. Each row is provided with three windows. The sample is dropped on the first sample pad 12 and the second sample pad 22 using a dropper or the first sample pad 12 and the second sample pad 22 are immersed in the sample for testing. The pen-shaped kit can realize loading samples separately, thus the first test paper strip 1 and the second test paper strip 2 do not interfere with each other.

### Example 10

Example 10 provides a method for detecting human pregnancy time by using the hCG cycle test paper kit of example 5. The test starts on the day when menstruation should come but not. If test starts in advance, test should be done again on the expected menstrual day. If menstruation is delayed, test should be done again after 3 days. If a menstrual cycle is irregular, the longest cycle in the most recent month should be calculated before the test. If a user doesn't know when her menstrual cycle is expected to start, test should be done on at least 19 days after your last sexual life without protection. The test is carried out by the following specific steps: (1) a tested sample, a kit and other equipment are equilibrated at room temperature; (2) an outer packaging of the kit is removed, and the test sample or reference sample are marked on the kit; (3) the sample pad of the first test paper strip and the sample pad of the second test paper strip of the kit are inserted into an urine sample, as shown in Fig. 4, the first sample pad 12 and the second sample pad 22 need to be completely immersed in the urine; (4) the first sample pad 12 and the second sample pad 22 are took out after 3-10 seconds and lay flat. Results of the color depths of the first detection line T1 and the second detection line T2 should be observed within 3-5 minutes, and they will be invalid after 5 minutes.

Based on the color depth of the first detection line T1 and the second detection line T2, the number of weeks of pregnancy can be estimated, and the time of conception can be estimated. The test results are as follows: (1) negative (-): only quality control lines C1 and C2 develop color, and the first detection line T1 and the second detection line T2 do not develop color, indicating non-pregnant; (2) positive (+): quality control lines C1 and C2 are always develop color; the colors of the first detection line T1 and the second detection line T2 are shown in the following table 1:

**Table 1: Interpretation of test results**

| Results | Pregnancy time | Pregnancy time determined by doctor (28-day of menstrual cycle) |
|---|---|---|
| First detection line Tldevelops color, second detection line T2 does not develop color | Test result is "pregnant"(+) and having a pregnancy time of about 1-2 weeks | 3-4 weeks |
| Second detection line T2 develops lighter color than first detection line T1 | Test result is "pregnant"(+) and having a pregnancy time of about 2-3 weeks | 4-5 weeks |
| Second detection line T2 develops lighter or closer to first detection line T1 | Test result is "pregnant"(+) and having a pregnancy time of more than 3 weeks | above 5 weeks |

(3) Invalid: if neither the detection T area (the first detection line T1 and the second detection line T2) and the control C area (the first quality control line C1 nor the second quality control line C2) shows prunosus line, or the control C area (quality control line) of the test paper strip doesn't show prunosus line, indicating that the test paper strip is invalid.
(4) Based on the pregnancy time suggested, you can estimate when to conceive. However, the doctor calculates the time of pregnancy based on the first day of the last menstrual cycle.

The present application uses the principle of double antibody sandwich method and immunochromatography to qualitatively detect human chorionic gonadotropin in urine, indicating the pregnancy cycle, and having relatively simple detection step, and the results can be observed within 3-5 minutes, and general users can test by yourself, the test results are more accurate. According to the present invention, the color depth of the first detection line and the second detection line can be used to estimate the number of weeks of pregnancy and to estimate the time of pregnancy.

### Experimental example 1

Random sampling is preformed from the same batch of samples with a minimum sampling volume not less than 3 times of the testing volume as required.

After sampling, the testing sample is placed in the yellow area to be tested. If it can be tested on that very day, it will be tested on the that very day. If it cannot be tested on that very day, the test must be completed the next day. The paper strips that pass the test can be temporarily placed in the qualified product area as a reserve sample, and then a certain quantity of products are taken from the production line for the reserve sample of the product according to the output and reserve sample requirements.
1. The required reagents are as follows:
   0 mIU/ml sample solution: protein-containing phosphate buffer (PBS) is 0 mIU/ml of the HCG sample solution, where the protein in the protein-containing phosphate buffer is bovine serum albumin accounting for 0.5% -1% by weight of the phosphate buffer.
   50m IU/ml, 25 mIU/ml, 12.5 mIU/ml human chorionic gonadotropin (hCG) sample solution prepared by human chorionic gonadotropin (hCG) standard and the protein-containing phosphate buffer (0.01M PBS) to reach a corresponding concentration respectively.
   400m IU/ml, 200 mIU/ml, 100 mIU/ml human chorionic gonadotropin (hCG) sample solution prepared by human chorionic gonadotropin (hCG) standard and the protein-containing phosphate buffer (PBS) to reach a corresponding concentration respectively.
   500m IU/ml human luteinizing hormone (hLH): After hLH standard is re-dissolved with the protein-containing phosphate buffer (PBS), 0m IU/ml HCG sample solution is used to prepare hLH A solution with a concentration of 500m IU/ml; 25m IU/ml human chorionic gonadotropin sample solution is used to prepare hLH B solution with a concentration of 500m IU/ml; 200m IU/ml human chorionic gonadotropin sample solution is used to prepare hLH C solution with a concentration of 500m IU/ml.
   1000m IU/ml human follicle stimulating hormone (hFSH): After the hFSH standard is re-dissolved with the protein-containing phosphate buffer (PBS), 0m IU/ml HCG sample solution is used to prepare hFSH A solution with a concentration of 1000m IU/ml; 25m IU/ml human chorionic gonadotropin sample solution is used to prepared hFSH B solution with a concentration of 1000m IU/ml; the human chorionic gonadotropin sample solution is used to prepare hFSH C solution with a concentration of 1000m IU/ml.
   1000µ IU/ml human thyroid stimulating hormone (hTSH): After the hTSH standard is re-dissolved with the protein-containing phosphate buffer (PBS), 0m IU/ml HCG sample solution is used to prepare hTSH A solution with a concentration of 1000µ IU/ml; 25m IU/ml human chorionic gonadotropin sample solution is used to prepare hTSH B solution with a concentration of 1000µ IU/ml; 200m IU/ml human chorionic gonadotropin sample solution is used to prepare hTSH C solution with a concentration of 1000µ IU/ml .
2. Physical properties:
   Testing appearance properties: The test paper strip is taken out and observed with eyesight. The paper strip should be neat and complete, have no burrs, no damage, and no pollution, and be firmly attached with the material. If it is a test pen, card or stick, it is also necessary to check whether it is assembled correctly.
   Film strip width: paper strips for three people are took out to measure the width of the film on the strips with a ruler. Chromatographic status: regular, the colloid gold not layered with water, and a background is basically clear within 3-5 minutes.
3. The minimum detectable quantity of the test: a national human chorionic gonadotropin standard is prepared into standard solutions with concentrations of 12.5 mIU/ml, 25 mIU/ml, and 50 mIU/ml. The samples for three people are extracted and tested. he results are observed within 3-5 minutes, showing that the minimum detectable quantity of the first detection line is 25 mIU/ml. Furthermore, a national human chorionic gonadotropin standard is prepared into standard solutions with concentrations of 100 mIU/ml, 200 mIU/ml, and 400 mIU/ml. The samples for three people are extracted and tested. The results are observed within 3-5 minutes, showing that the minimum detectable quantity of the second detection line is 100 mIU/ml.
4. Specificity:
   Negative specificity: 500m IU/ml hLH A solution, 1000m IU/ml hFSH A solution, 1000µ IU/ml hTSH A solution are tested for 3 times respectively. If results of the three times of each solution show that both of the first and second detection lines are negative, it can be determined that the result of this solution is negative.
   Positive specificity: 500m IU/ml hLH B solution, 1000m IU/ml hFSH B solution, and 1000µ IU/ml hTSH B solution are tested for 3 times respectively. Results of the three times of each solution show that the first detection line is positive, and the second detection line does not develop color, which is negative. 500 mIU/ml hLH C solution, 1000 mIU/ml hFSH C solution, and 1000 µIU/ml hTSH C solution are tested for 3 times respectively. Results of the three times of each solution show that both of the first and second detection lines are positive, and the color of the second detection line is significantly lighter than that of the first detection line.
5. Repeatability:
   10 strips of the same batch of test strips are randomly selected and measured with a standard of 25 mIU/ml human chorionic gonadotropin. The results of 10 times show that all of first detection lines are positive and have color with a consistent intensity, and the second detection lines do not develop color and are negative. 10 strips of the same batch of test strips are randomly selected and measured with a standard of 200 mIU/ml human chorionic gonadotropin. The results of 10 times show that the first and second detection lines are positive, and the color of the second detection line is significantly lighter than that of the first detection line.
6. Stability:
   The test paper strips placed at 37 °C for 21 days or after one month from the expiration date of the product are tested according to requirements of items 2, 3, 4, and 5. The results should meet the requirements.
7. Difference between batches:
   3 batches of test paper strips are tested with 25 mIU/ml human chorionic gonadotropin standard for 10 times. The results show that all of the first detection lines are positive and develop consistent color, and the second detection lines do not develop color and are negative. 3 batches of test papers are tested with 200 mIU/ml human chorionic gonadotropin standard for 10 times. The results show that the first detection line and the second detection line should be positive, and the color of the second detection line is significantly lighter than that of the first detection line.
8. Hook effect: when the hCG concentration exceeds 50,000 mIU/ml, a hook effect may occur.

Human chorionic gonadotropin cycle test paper is used to qualitatively detect human chorionic gonadotropin (hCG) in urine of pregnant women, suggesting the pregnancy cycle.

Human chorionic gonadotropin (hCG) is a glycoprotein hormone secreted by placental trophoblast cells during pregnancy. It consists of two different subunits, α and β connected by a non-covalent bond. During production, secretion, and metabolism of hormones, hCG molecules will undergo various changes such as rupture and dissociation, so as to exist in various molecular forms in blood and urine. hCG is the only placenta hormone that does not increase with the increase in placental weight. A concentration of hCG is 5-50 mmIU/ml in the blood and >25 mIU/ml in the urine after 1 week of pregnancy, reaches a peak around 8-10 weeks, and decreases rapidly after the next 1-2 weeks, and then gradually decreases and maintains at about 1/5-1/10 of the peak level until delivery. The present application uses the principle of double antibody sandwich method and immunochromatography to qualitatively detect human chorionic gonadotropin in urine, indicating the pregnancy cycle, having relatively simple detection step, and the results can be observed within 3-5 minutes, and general users can test by yourself, the test results are more accurate. According to the present invention, the color depth of the first detection line and the second detection line can be used to estimate the number of weeks of pregnancy and to estimate the time of pregnancy.

It is apparent that the above embodiments are merely examples for clarity of illustration, and are not intended to limit the embodiments. Other variations or modifications in various forms may be made by those skilled in the art in view of the above description. There is no need and no way to present all of the embodiments herein. The obvious variations or modifications derived therefrom are still within the scope of protection of the present application.

## Claims

1. A hCG cycle test paper strip, **characterized by** comprising a first test paper strip and a second test paper strip,
wherein the first test paper strip and the second test paper strip respectively comprise:
a substrate, and
a sample pad, a colloidal gold adsorption pad, an antibody carrying film and a water absorption pad sequentially adhered to the substrate;
wherein the sample pad, the colloidal gold adsorption pad, the antibody carrying film, and the water absorption pad are only in contact with and only partially overlap with adjacent pad thereof; the antibody carrying film has a quality control line and a detection line arranged at an interval, the quality control line is close to the water absorption pad, and the detection line is close to the colloidal gold adsorption pad;
colloidal gold-anti-human chorionic gonadotropin β-hCG monoclonal antibody conjugate is adsorbed in the colloidal gold adsorption pad of the first test paper strip, and colloidal gold-anti-human chorionic gonadotropin β-hCG monoclonal antibody conjugate and a free β antibody are absorbed in the colloidal gold adsorption pad of the second test paper strip;
the detection line is coated with an anti-human chorionic gonadotropin α-hCG monoclonal antibody; the quality control line is coated with an anti-mouse IgG polyclonal antibody.

2. The hCG cycle test paper strip according to claim 1, **characterized in that**, the antibody carrying film is a nitrocellulose film having a pore size of 3-10 um.

3. The hCG cycle test paper strip according to claim 1 or 2, **characterized in that**, the sample pad and the water absorption pad are each provided with a protective film.

4. A method for preparing a hCG cycle test paper strip according to any one of claims 1 to 3, **characterized by** comprising the steps of:
S10: coating an antibody carrying film with a detection line and a quality control line disposed at an interval of 0.3-0.7cm in parallel, and drying at a constant temperature of 4-35 °C;
S20: immersing the antibody carrying film obtained in step S10 in PBS buffer, and then immersing in a blocking treatment solution, and drying at a temperature of 18-28 °C and a relative humidity of ≤40%;
S30: preparing a colloidal gold complex solution and adjusting pH to 6.5-7.0, adding anti-human chorionic gonadotropin β-hCG monoclonal antibody, and then adding a stabilizer to mix well, centrifuging and collecting a precipitate, using the colloidal gold complex solution to re-dissolve the precipitate, casting on a colloidal gold adsorption pad of a first test paper strip, and followed by drying, sealing and storing;
S40: preparing a colloidal gold complex solution and adjusting pH to 6.5-7.0, adding anti-human chorionic gonadotropin β-hCG monoclonal antibody, and then adding a stabilizer to mix well, centrifuging and collecting a precipitate, using the colloidal gold complex solution to reconstitute the precipitate, and then adding free β antibody to mix well, casting on a colloidal gold adsorption pad of a second test paper strip, and followed by drying, sealing and storing;
S50: adhering a sample pad, the colloidal gold adsorption pad of the first test paper strip, the antibody carrying film, and a water adsorption pad sequentially along a length direction of a substrate to obtain a first test paper strip; and adhering the colloidal gold adsorption pad of the second test paper strip, the antibody carrying film, and a water adsorption pad sequentially along a length direction of a substrate to obtain a second test paper strip.

5. The method according to claim 4, **characterized in that**, a concentration of anti-human chorionic gonadotropinα-hCG monoclonal antibody used for coating the detection line is 1.1-1.5 mg/mL, preferably 1.3 mg/mL; a concentration of anti-mouse IgG polyclonal antibody used for coating the quality control line is 1.5-2.5 mg/mL, preferably 2 mg/mL.

6. The method according to claim 4 or 5, **characterized in that**, the blocking treatment solution comprises 0.08-0.12Mol buffer solution, 0.3-0.7wt% sugar, and 0.8-1.2wt% blocking protein, and 0.03-0.08wt% preservative, preferably 0.1Mol buffer solution, 0.5wt% sugar, 1wt% blocking protein, and 0.05wt% preservative.

7. The method according to claim 6, **characterized in that**, the buffer solution is phosphate buffer, the sugar is sucrose or trehalose, the preservative is NaN₃ or thimerosal, and the blocking protein is casein or bovine serum albumin.

8. The method according to any one of claims 4 to 7, **characterized in that**, a concentration of the anti-human chorionic gonadotropin β-hCG monoclonal antibody in the step S30 is 5 µg/mL.

9. The method according to any one of claims 4 to 8, **characterized in that**, a concentration of the anti-human chorionic gonadotropin β-hCG monoclonal antibody in the step S40 is 5 µg/mL, and a weight ratio of the free β antibody to the anti-human chorionic gonadotropin β-hCG monoclonal antibody is 10: 1.

10. The method according to any one of claims 4 to 9, **characterized in that**, the colloidal gold-anti-human chorionic gonadotropin β-hCG monoclonal antibody conjugate is cast on the colloidal gold adsorption pad of the first test paper strip with a casting amount of 50 µl/cm²;
the colloidal gold-anti-human chorionic gonadotropin β-hCG monoclonal antibody conjugate and the free β antibody are cast on the colloidal gold adsorption pad of the second test paper strip with a casting amount of 50 µl/cm².

11. A hCG cycle test kit, **characterized by** comprising a hCG cycle test paper strip according to any one of claims 1-3 or a hCG cycle test paper strip prepared by the method according to any one of claims 4-10.

12. The hCG cycle test kit according to claim 11, **characterized by** further comprising a casing, wherein the casing is provided with two grooves arranged in parallel and used for placing the first test paper strip and the second test paper strip, respectively.

13. The hCG cycle test kit according to claim 12, **characterized by** further comprising a cover body, wherein the cover body is provided with two parallel windows and detachably covered on the casing, and the detection line and quality control line of the first test paper strip and the second test paper strip can be observed through the windows.

14. Use of the hCG cycle test paper strip according to any one of claims 1-3 or the hCG cycle test paper strip prepared by the method according to any one of claims 4-10 or the hCG cycle test kit according to any one of claims 11-13 in detecting human pregnancy time.

15. A method for detecting human pregnancy time by using the hCG cycle test paper strip according to any one of claims 1-3, **characterized by** comprising the steps of:
a) taking urine of a person to be tested, and then completely soaking sample pads of the first test paper strip and the second test paper strip into the urine for 3-10 seconds, and then taking the sample pads out and laying flat, observing a result within 3-5 minutes, the result is invalid after 5 minutes;
b) observing color depth of detection lines T1 and T2 of the first test paper strip and the second test paper strip and whether quality control line C1 of the first test paper strip and quality control line C2 of the second test paper strip are colored or not:
if quality control lines C1 and C2 develop color, and detection lines T1 and T2 do not develop color, it indicates that the tested person is not pregnant;
if quality control lines C1 and C2 develop color, and detection line T1 develops color, and detection line T2 does not develop color, it indicates that the tested person is pregnant and has a pregnancy time of 1-2 weeks;
if quality control lines C1 and C2 develop color, detection line T1 develops color, detection line T2 develops lighter color than detection line T1, it indicates that the tested person is pregnant and has a pregnancy time of 2-3 weeks;
if quality control lines C1 and C2 develop color, detection line T1 develops color, detection line T2 develops lighter or closer to detection line T1, it indicates that the tested person is pregnant and has a pregnancy time of more than 3 weeks; and
if neither quality control lines C1 and C2 nor detection lines T1 and T2 show prunosus, or no quality control lines C1 and C2 show prunosus, it indicates that the hCG cycle test paper strip is invalid.
